# EUROPEAN PATENT APPLICATION

(11) **EP 1 052 319 A1**
(43) Date of publication of application: **15.11.2000**
(21) Application number: 00303336.2
(22) Date of filing: 19.04.2000
(51) Int. Cl.: D04B 21/02

(54) **Three-dimensional composite fabric articles**

(30) Priority: 03.05.1999 US 303856
(71) Applicant: MALDEN MILLS INDUSTRIES, INC., Lawrence, Massachusetts 01842 (US)
(72) Inventor: Rock, Moshe, Andover, MA 01810 (US); Sharma, Vikram, Stoneham, MA 02180 (US); Vainer, Gadi, Melrose, MA 02176 (US)
(74) Representative: Colmer, Stephen Gary

(57) **Abstract**

A three-dimensional fabric (12) article includes a first fabric layer (14) made from a first stitch yarn (16) and a first backing yarn (24), a second fabric layer (18) made from a second stitch yarn (20) and a second backing yarn (26), an interconnecting yarn (22) resiliently connecting the first and second fabric layers, and an elastomeric yarn incorporated into at least one of the first stitch yarn (16), the first backing yarn (24), the second stitch yarn (20), and the second backing yarn (26). The three-dimensional fabric article can be included in a medical therapeutic appliance, wherein at least one of the fabric layers has a raised surface compatible for releasable, secure engagement by a hook component of a hook-and-loop type fastening system.

## Description

This application is a continuation-in-part of U.S. Application No. 09/204,524, filed December 10, 1998, which is a continuation of U.S. Application No. 08/839,297, filed April 17, 1997.

The invention relates to three-dimensional composite fabric structures having first and second fabric layers connected to each other.

### Background of the Invention

It is known to employ compression as a therapeutic aid for certain injuries, with constant pressure being desired in all directions. Closed cell foams of resilient synthetic resin, such as neoprene, are generally used in these devices. Stretch knits and stretch woven fabrics are also employed, but by themselves are not able to provide desired insulation and compression. Neoprene fabrics have relatively rough surface aesthetics, and neoprene has a pungent, irritating odor and permits only low levels of moisture vapor transmission. Neoprene is also particularly known to provide very little air permeability, thus reducing its ability to breathe and reducing comfort for the wearer. Also, for compatibility with hook components of hook-and-loop-type releasable fasteners, such as available from Velcro Industries, B.V., of Manchester, New Hampshire, under the trademark VELCRO®, neoprene products have to be laminated at one or both surfaces with special loop fabric elements, e.g. as available commercially, but the laminated neoprene products have only unidirectional stretch. Alternatively, neoprene products are laminated with stretch jersey knit fabrics on both surfaces, face and back.

Three dimensional composite fabric structures are described, e.g., in Rock et al. U.S. Patent No. 5,783,277, issued July 21, 1998, and U.S. Patent Application No. 08/839,297, filed April 17, 1997, the complete disclosures of which are incorporated herein by reference.

### Summary of the Invention

According to one aspect of the invention, a three-dimensional fabric article comprises a first fabric layer made from a first stitch yarn and a first backing yarn, a second fabric layer made from a second stitch yarn and a second backing yarn, an interconnecting yarn resiliently connecting the first and second fabric layers, and an elastomeric yarn incorporated into at least one of the first stitch yarn, the first backing yarn, the second stitch yarn, and the second backing yarn.

Preferred embodiments of this aspect of the invention may include one or more of the following additional features. One or both of the first and second fabric layers has a raised surface, preferably created by at least one of napping, sanding and brushing. The interconnecting yarn is a multi-filament yarn or a combination multi-filament, preferably texturized, and monofilament yarn. The first fabric layer, i.e., at least one of the first stitch yarn, the first backing yarn, and the interconnecting yarn, has been rendered hydrophilic. The second fabric layer is hydrophobic. The elastomeric yarn is an elastomeric wrap yarn or an elastomeric bare yarn. The three-dimensional fabric article further comprises an additional fabric layer attached, e.g. laminated, upon the second fabric layer. Preferably, the additional fabric layer is treated for water repellency.

According to another aspect of the invention, a medical therapeutic appliance comprises a three-dimensional fabric article comprising a first fabric layer made from a first stitch yarn and a first backing yarn, a second fabric layer made from a second stitch yarn and a second backing yarn, an interconnecting yarn resiliently connecting the first and second fabric layers, and an elastomeric yarn incorporated into at least one of the first stitch yarn, the first backing yarn, the second stitch yarn and the second backing yarn, at least one of the fabric layers having a raised surface compatible for releasable, secure engagement by a hook component of a hook-and-loop type fastening system.

Preferred embodiments of this aspect of the invention may include one or more of the following additional features. The first and second fabric layers have raised surfaces, preferably created by at least one of napping, sanding and brushing. The first fabric layer has been rendered hydrophilic and the second fabric layer is hydrophobic. The three-dimensional fabric article further comprises at least one hook component of a hook-and-loop type fastening system, adapted for releasable, secure engagement with the raised surface.

The invention thus provides a three-dimensional composite fabric article that has high moisture vapor transmission, capability of stretch, and recovery, with compressible performance features, and a method of making the same. One or both surfaces of the composite structure can, with proper yarn selection, be finished, e.g. by napping, sanding, or brushing, to render one or both surfaces compatible for releasable, secure engagement by hook components of a hook-and-loop type fastening system. The resulting three dimensional composite fabric article is stretchable, compressible with air permeability and high moisture vapor transmission, breathable, and insulating, and it has application to any of various end uses, such as in medicine, footwear or outerwear, and in any other related fields of use requiring such properties.

Objectives of the invention include to provide a three-dimensional composite fabric structure with the following characteristics: ability to breathe, bi-directional stretch and recovery, compressibility, compatibility with hook components of commercial hook-and-loop type releasable fasteners, and capability of lamination with other fabrics, as desired.

Other objectives of the invention include to provide an improved knit fabric construction for enhanced shape retention; to provide a fabric having improved moisture vapor transmission; to provide a fabric having improved stretch and recovery after stretching; to provide a fabric which allows only a limited amount of air passage; to provide a fabric with high heat retention and insulation properties; and to provide a fabric having one or both surfaces compatible for releasable, secure engagement by a hook component of a hook-and-loop type releasable fastener.

Other features and advantages of the invention will be apparent from the following description of a presently preferred embodiment, and from the claims.

### Brief Description of the Drawings

Fig. 1 is a somewhat diagrammatic perspective view showing the structure of a three-dimensional composite fabric article of the invention; and
Fig. 2 is a somewhat diagrammatic side view of the three-dimensional composite fabric structure of Fig. 1.
Fig. 3 is a somewhat diagrammatic side view of one embodiment of three-dimensional composite fabric article of the invention.
Fig. 4 is a somewhat diagrammatic side view of another embodiment of three-dimensional composite fabric article of the invention.
Figs. 5 and 6, and Fig. 7, are somewhat diagrammatic perspective views of alternate arrangements of medical therapeutic compression devices of the invention.

### Detailed Description of the Invention

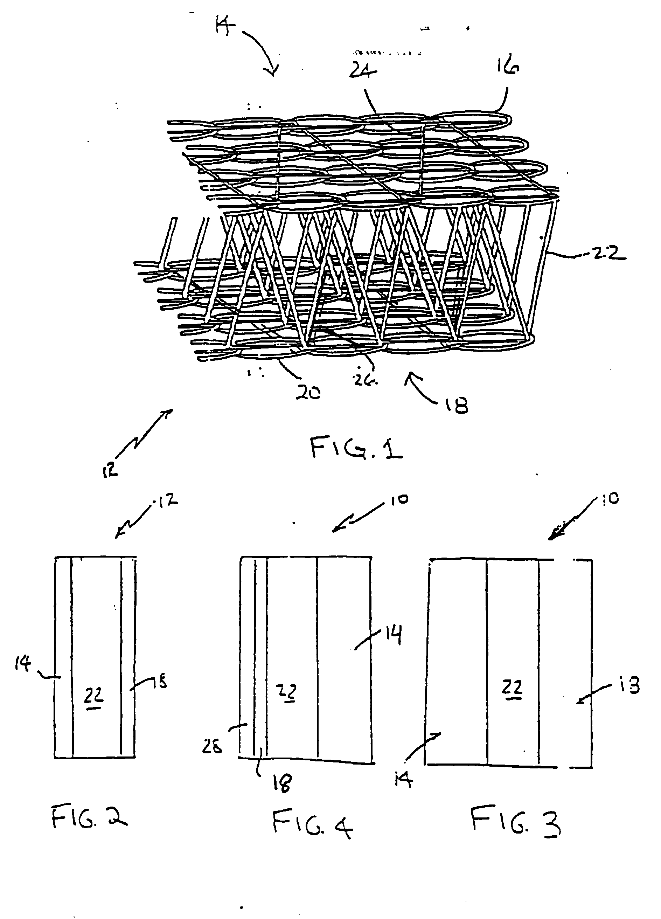

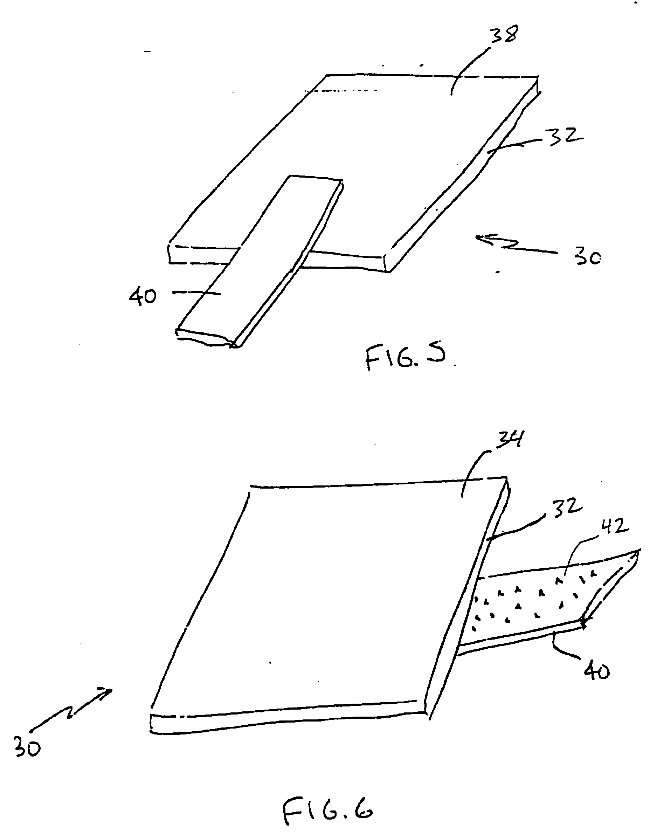

A three-dimensional composite fabric article 10 of the invention (Figs. 3 and 4) is prepared by knitting a three-dimensional composite fabric structure 12 (Figs. 1 and 2) on a double-needle bar warp knitting machine commonly used in the manufacture of velvet and well known in the art, while controlling the bulk ratio of the stitch and interconnecting yarns.

Referring to Figs. 1 and 2, the three-dimensional composite fabric structure 12 has an inside (or first or top) fabric layer 14 made from stitch yarn 16, an outside (or second) fabric layer 18 made from stitch yarn 20, and interconnecting yarn 22 connecting the two layers 14, 18. In addition, the composite fabric structure 12 typically includes backing or lay-in yarns 24, 26 held by stitch yarns 16, 20, respectively. One or more of the stitch yarns 16, 20 and lay-in yarns 24, 26 incorporates an elastomeric wrap yarn or an elastomeric bare yarn, i.e. spandex, such as available from E.I. duPont de Nemours & Company, Inc., of Wilmington, Delaware, under the trademark LYCRA®.

The yarns of the three-dimensional composite fabric structure 12 of the invention are preferably a synthetic material such as polyester, acrylic or nylon, or a combination thereof. The yarns may be filament or spun, texturized or fully oriented.

The interconnecting yarn 22 connecting the two layers 14, 18 of the three-dimensional composite fabric structure 12 is selected to have resilience and stiffness sufficient to urge the fabric layers 14, 18 apart, e.g., when pressure is applied to the surface of either of the fabric layers. In construction, the interconnecting yarn 22 may be made of the same material as any of the fabric layer yarns, or it may be made of a different material. Preferably, the interconnecting yarn 22 is made of a resilient material such as a multi-filament yarn, or a combination multi-filament and monofilament yarn, of polyester, nylon, or the like.

The composite fabric article 10 is designed to facilitate moisture transport away from the wearer's body, while providing an inside layer surface, i.e., the surface forming the surface next to the user's skin, which is comfortable and maintains air circulation next to the skin. For this purpose, any one or more of the yarns of inside layer 14 is made from fibers rendered hydrophilic to facilitate transport of moisture through the surface, away from the skin. Typically, the coarsest yarn, which generally is or includes the interconnecting yarn, is rendered hydrophilic, since the yarn with the greatest coarseness, i.e. thickness or bulk, has the most loft or height from the surface of the fabric, bringing it most into contact with the wearer's skin. As discussed further below, the yarn with greatest coarseness is also typically easiest to access for finishing, e.g. to create fleece or the like, thus bringing this yarn still more into contact with the wearer's skin.

In one preferred embodiment, inside fabric layer 14 is made from a stitch yarn 16 having a fineness of between about 50 and 200 denier, and preferably about 100 denier, with an individual fiber fineness in the range of between about 0.5 and 10.0 dpf (denier per fiber), and preferably about 3.0 dpf. The backing or lay-in yarn 24 of inside layer 14 is multi-filament, with a fineness of between about 50 and 300 denier, and preferably about 150 denier, and with an individual fiber fineness in the range of between about 0.5 and 10.0 dpf, and preferably about 2.0 dpf.

Preferably, stitch yarn 16, backing yarn 24 and/or interconnecting yarn 22 of first fabric layer 14 are made of polyester or nylon that has been rendered hydrophilic in order to enhance the transport of moisture, e.g. perspiration, thereby to help keep the surface of the wearer's skin beneath the composite fabric article 10 dry and comfortable. Particularly, layer 14 is chemically treated or utilizes modified fibers so that it is rendered hydrophilic, e.g. as described in Lumb et al. U.S. Patent No. 5,312,667, the entire disclosure of which is incorporated herein by reference.

By using a chemically modified fiber or by chemically treating layer 14, the layer is rendered substantially hydrophilic. As a result, the transport of perspiration from the surface is substantially enhanced, especially if the surface of layer 14 is raised, as described below, so that liquid moisture is made readily transportable along the surface of layer 14.

Interconnecting yarn 22, which connects the two layers 14, 18, is a multi-filament yarn, or a combination multi-filament, preferably texturized, and monofilament yarn, the multi-filament yarn having a fineness of between about 100 and 600 denier, and preferably about 300 denier, and having an individual fiber fineness in the range of about 0.5 to 5.0 dpf, and preferably about 2.0 dpf. For relatively greater resilience, however, it is preferred that interconnecting yarn 22 is a combination multi-filament, preferably texturized, and monofilament yarn. The interconnecting yarn is typically made from fiber rendered hydrophilic in order to further facilitate transport of moisture from inside layer 14 to outside layer 18. Moreover, interconnecting yarns 22 are spaced from one another in a manner to allow air flow throughout the body of the fabric article 10. This improves cushioning, ventilation, and moisture vapor transmission.

Outside fabric layer 18 is made from stitch yarn 20 having a fineness of about 50 to 200 denier, and preferably about 100 denier, with an individual fiber fineness in the range of about 0.5 to 10.0 dpf, and preferably about 3.0 dpf. Backing or lay-in yarn 26 has a fineness of between about 50 to 300 denier, and preferably about 150 denier, with an individual fiber fineness in the range of about 0.5 to 10.0 dpf, and preferably about 2.0 dpf.

Stitch yarn 20 and backing yarn 26 are multi-filament or monofilament, with a high tenacity value in order to increase toughness. In particular, each of the yarns 20, 26 has a tenacity of between about 3 grams and 12 grams per denier. This level of tenacity is selected to improve abrasion-, tear-, and rupture-resistance of fabric layer 18, e.g. in embodiments where these features are desired.

The composite fabric article 10 of the invention also incorporates an elastomeric yarn, i.e. spandex, such as available from E.I. duPont de Nemours & Company, Inc. under the trademark LYCRA®, in one or both of lay-in yarns 24, 26 of layers 14, 18, respectively. Such elastomeric yarn has a total fineness of between about 70 and 300 denier of spandex. This enhances the softness and flexibility of the layers, and the tightness of fit to the wearer. The stitch yarns 16, 20 of one or both of the fabric layers 14, 18 may also incorporate elastomeric yarns.

Referring to Fig. 3, the surface of one, or, preferably, both of the fabric layers 14, 18 (as shown) is finished, e.g., sanded, brushed or napped, to create a raised surface fabric, with each fiber end being a conductor of moisture. Fabric layer 14, after finishing, includes a plurality of fibers for conducting perspiration therealong, away from the skin of the wearer, and eventually to the second fabric layer 18, from where the moisture is evaporated. Fabric layer 18, after finishing, also includes a plurality of fibers for speeding the rate of moisture evaporation.

Alternatively, in another embodiment, shown in Fig. 4, only the surface region of fabric layer 14 is finished, and the unfinished fabric layer 18 is laminated (using any known, suitable commercial lamination technique, e.g. gravure roll, hot melt, flame lamination, etc.) with a stretchable laminating fabric 28, e.g. jersey or tricot. The adhesive system can be water based, solvent based or hot melt type, and the adhesive can be urethane, acrylic, polyester or any other thermoplastic type. The laminating fabric provides increased abrasion resistance and durability, especially to pilling, and/or may be selected for general aesthetics. Preferably, the laminating fabric 28 is treated for water repellency, to provide further protection. The resulting composite fabric article is stretchable, compressible, and breathable, and it can be used for medical end uses, e.g. as an orthopaedic support, or in any other related field of use requiring such properties.

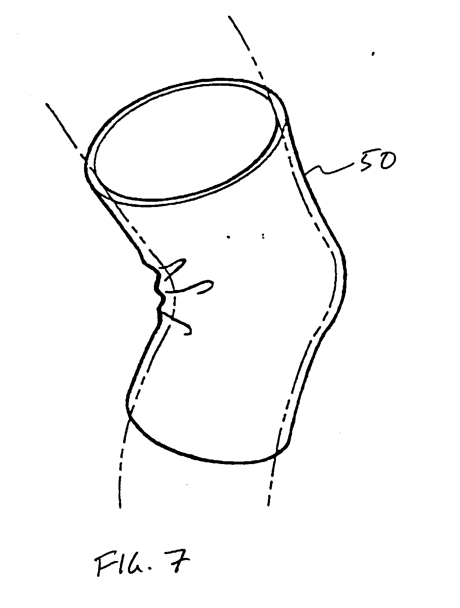

Referring to Figs. 5 and 6, a medical orthopaedic support apparatus 30 of the invention, such as previously formed of neoprene synthetic rubber or the like, includes a three-dimensional knit fabric article 32 of the invention having an inside surface 34 and/or an outside surface 38 finished to create a nap or fleece, i.e, raised, surface, and a releasable fastener 40. The releasable fastener 40 has a multi-hook surface 42 (Fig. 6), e.g. as used in a cooperative hook-and-loop type releasable fastening system, such as available under the trademark VELCRO®. The raised nap or fleece surfaces 34 (Fig. 5) and/or 38 (Fig. 6) are compatible for secure but releasable engagement with the multi-hook surface 42. It has also been found that the level of secureness, or holding power, with which the surfaces 34 and/or 38 are engaged by hook components of the hook-and-loop type fastener may be enhanced, i.e. increased, by employing yarns in the position to be finished having one or more of the characteristics of greater coarseness, higher crimp, higher stretchability, greater bulk, and shorter height of raised surface.

Referring to Fig. 7, another medical orthopaedic support apparatus 50 of the invention, e.g. a brace for the knee, is shown.

The three-dimensional composite fabric article 10 of the invention is compressible with great stretch and recovery performance. It inherently maintains its shape with a high degree of moisture vapor permeability. Use of texturized interconnecting yarn 22 connecting the two fabric surface layers 14, 18 provides very high insulation and thermal properties, which are therapeutic in medical end uses, e.g. in orthopaedic support fabrics. In a described preferred embodiment, the surface of the inner layer 14 is napped and the material of inner layer 14 is chemically treated to render the inner surface hydrophilic, thus making the inner surface a moisture transporting surface. The outer layer 18 is hydrophobic in this embodiment (where desired, it may instead be neutral or hydrophilic), thus to resist any external water from coming through the surface, but to allow moisture from the inner layer 14 to pass outward to evaporate. These are very desirable features in therapeutic applications, since sweat is not allowed to accumulate and create discomfort to the wearer of such orthopaedic support fabrics.

Other embodiments are within the scope of the following claims.

## Claims

1. A three-dimensional fabric article comprising:
a first fabric layer made from a first stitch yarn and a first backing yarn,
a second fabric layer made from a second stitch yarn and a second backing yarn,
an interconnecting yarn resiliently connecting said first fabric layer and said second fabric layer, and
an elastomeric yarn incorporated into at least one of said first stitch yarn, said first backing yarn, said second stitch yarn, and said second backing yarn.

2. The three-dimensional fabric article of claim 1, wherein said first fabric layer has a raised surface.

3. The three-dimensional fabric article of claim 2, wherein said raised surface of said first fabric layer is created by at least one of napping, sanding and brushing.

4. The three-dimensional fabric article of claim 1 or 2, wherein said second fabric layer has a raised surface.

5. The three-dimensional fabric article of claim 4, wherein said raised surface of said second fabric layer is created by at least one of napping, sanding and brushing.

6. The three-dimensional fabric article of claim 1, wherein said interconnecting yarn is a multi-filament yarn.

7. The three-dimensional fabric article of claim 1, wherein said interconnecting yarn is a combination multi-filament and monofilament yarn.

8. The three-dimensional fabric article of claim 1, 6 or 7, wherein said interconnecting yarn is texturized.

9. The three-dimensional fabric article of claim 1, wherein said first fabric layer has been rendered hydrophilic.

10. The three-dimensional fabric article of claim 9, wherein at least one of said first stitch yarn, said first backing yarn, and said interconnecting yarn has been rendered hydrophilic.

11. The three-dimensional fabric article of claim 1 or 6, wherein said second fabric layer is hydrophobic.

12. The three-dimensional fabric article of claim 1, wherein said elastomeric yarn is an elastomeric wrap yarn.

13. The three-dimensional fabric article of claim 1, wherein said elastomeric yarn is an elastomeric bare yarn.

14. The three-dimensional fabric article of claim 1, further comprising an additional fabric layer attached upon said second fabric layer.

15. The three-dimensional fabric article of claim 14, wherein said additional fabric layer is laminated upon said second fabric layer.

16. The three-dimensional fabric article of claim 14, wherein said additional fabric layer is treated for water repellency.

17. A medical therapeutic appliance comprising a three-dimensional fabric article comprising a first fabric layer made from a first stitch yarn and a first backing yarn, a second fabric layer made from a second stitch yarn and a second backing yarn, an interconnecting yarn resiliently connecting said first fabric layer and said second fabric layer, and an elastomeric yarn incorporated into at least one of said first stitch yarn, said first backing yarn, said second stitch yarn, and said second backing yarn, at least one of said first fabric layer and said second fabric layer having a raised surface compatible for releasable, secure engagement by a hook component of a hook-and-loop type fastening system.

18. The three-dimensional fabric article of claim 17, wherein said first fabric layer has a raised surface and said second fabric layer has a raised surface.

19. The three-dimensional fabric article of claim 17 or 18, wherein said raised surface is created by at least one of napping, sanding and brushing.

20. The three-dimensional fabric article of claim 17, wherein said first fabric layer has been rendered hydrophilic and said second fabric layer is hydrophobic.

21. The three-dimensional fabric article of claim 17, further comprising at least one hook component of a hook-and-loop type fastening system, adapted for releasable, secure engagement with said raised surface.
